# EUROPEAN PATENT SPECIFICATION

(11) **EP 3 110 465 B1**
(45) Date of publication and mention of the grant of the patent: **18.04.2018**
(21) Application number: 15710849.9
(22) Date of filing: 25.02.2015
(51) Int. Cl.: A61M 1/00, A61M 39/22

(54) **ASPIRATORS**
ASPIRATOR
ASPIRATEUR

(30) Priority: 28.02.2014 GB 201403597
(43) Date of publication of application: 04.01.2017
(73) Proprietor: Aspirate N Go Ltd., Liverpool L3 5RF (GB)
(72) Inventor: GALLAGHER, George, Liverpool, L3 5RF (GB)
(74) Representative: Hutchinson, Thomas Owen
(86) International application number: PCT/GB2015/050538
(87) International publication number: WO 2015/128637

(56) References cited:
- EP-A1- 1 774 985
- WO-A1-87/06455
- WO-A1-96/17550
- GB-A- 2 185 689
- GB-A- 2 338 898
- JP-A- 2008 237 309

## Description

This invention relates to aspirators, and in particular, but without limitation, to aspirators suitable for drawing fluids from within a body cavity of a human or animal patient.

Aspirators are used in a range of medical procedures where fluids need to be drawn from within a body cavity, for diagnostic, sampling and/or therapeutic purposes.

EP1774985 discloses a catheter with a colour-changing element within it; comprising a syringe for drawing up fluid; a one-way valve; and a filter element configured to allow air and/or gas to flow through, but inhibit fluid.

WO9617550 discloses a sampling device for drawing fluids from a body; comprising a syringe for drawing up fluid; a fluid receiving receptacle; a filter element; and manual valves, such that the direction of flow may be manually altered.

GB2338898 discloses an aspiration kit for drawing pleural fluid from a body; comprising a syringe; a drainage bag; and a three-way tap, wherein the direction of flow is determined by the position of the tap.

JP2008237309 discloses a confirming member for identifying the positioning of a catheter tip; comprising a suction means; a colour-changing element; and a channel switching member.

GB21855689 discloses an apparatus for draining fluid from a body cavity; comprising an integral cannula; a pump means; a filter element; and a double check valve.

WO8706455 discloses ophthalmic aspirator for removing debris from the eye; comprising a needle; a syringe; a discharge opening; and two one-way valves, wherein the one way valves are configured to allow fluid to be discharged into the eye and permit fluid taken from the eye to pass into the discharge opening.

Pumped aspirators generally comprise a vacuum pump connected to a tube that can be inserted, or fed, into a body cavity, such that when the pump is switched on, the vacuum from the pump draws fluid from the body cavity through the tube, provided, of course, that the tip of the tube is located within the fluid to be aspirated. A liquid trap is usually interposed between the tube and the vacuum pump to prevent aspirated liquids from being drawn into the pump, which could damage and/or contaminate the pump. When using a vacuum pump aspirator, care needs to be taken to ensure that the vacuum is not too high and that the quantity and rate of aspiration is monitored. Monitoring and control circuitry can often be used to facilitate this, as well as the manual interventions of an experienced operator.

Where only relatively small amounts of fluids need to be aspirated, it is commonplace for a practitioner to use a syringe-based aspirator instead of a vacuum pump-based aspirator. In such a situation, the syringe is emptied (i.e. its plunger depressed) before being connected to the end of an aspirator tube. Prior to this, of course, the aspirator tube will have been inserted into the body cavity in a known manner, i.e. by feeding the tube with a guide wire inserted into it for rigidity, and upon location of the tip of the tube at a desired location, withdrawing the guide wire to leave the end of the tube extending from the body. When the aspirator tube has been correctly inserted, its tip will be submerged in the liquid to be aspirated. It should then be a relatively straightforward task to connect a pump or a syringe to the free end of the aspirator tube to aspirate the liquid. In the case of a syringe based aspirator, this is achieved by withdrawing the syringe's plunger, to create a vacuum in the tube, which vacuum can be used to aspirate the liquid. Generally speaking, syringes are sterile, inexpensive, disposable items, and so the problem of drawing aspirated fluids into the syringe is not usually a problem, and thus, there is usually no need to guard and/or proof the syringe against liquid ingress, i.e. to provide a fluid trap or any controls. In fact, in most cases, a simple syringe connected to a flexible tube is usually all that is required, as well as a skilled operator, of course.

Despite their relative simplicity and low-cost, known syringe-based aspirators suffer from a number of problems:

First, if a medical practitioner attempts to aspirate fluids (in particular liquids), especially relatively viscous fluids, through a relatively long pipe, an over-sized syringe needs to be used. The reason for this is that, due to the nature of the relationship between volume and pressure of air, in an isothermal expansion process, the volume of the syringe needs to be considerably larger than the internal volume of the tube to achieve a sufficient vacuum to draw fluids along the tube, especially against the effects of air pressure and gravity, which also act upon the fluid to be aspirated.

Second, viscous liquids exert a hydrodynamic drag and, unless the internal sidewalls of the aspirator tube are hydrophobic (by which is generally meant: aspirated liquid-phobic) the aspirated fluid sticks to the sidewalls of the tube leading to drag also. What this means is that the vacuum provided by the syringe needs to be greater than for a non-viscous liquid.

Third, the volume of air within the tube needs to be aspirated as well as the fluid itself. This can be counteracted, to an extent, by using a narrower bore tube, but narrower tubes tend to be more susceptible to clogging and pinching off, as well as increasing the hydrodynamic drag of a viscous aspirated liquid: thus a compromise needs to be found in this regard.

The upshot of the above, is that, in practice, practitioners tend to require a syringe whose volume is a factor of 10 to 100 or so greater than the volume of fluid to be aspirated (for example, a 50ml syringe for a drop of aspirated fluid, or a 100ml syringe for few ml of aspirated fluid). However, larger syringes are more awkward to handle, are more expensive to buy, more bulky to store and transport, and tend to be in shorter supply in medical environments than smaller syringes.

Nevertheless, in many instances, even a 100x over-sized syringe is insufficient to aspirate fluids (especially liquids) during a first attempt (the first withdrawal of the syringe plunger) and if the first attempt is unsuccessful, the practitioner, using a syringe-type aspirator, has two options:
Firstly, the practitioner can: 1) pinch-off the tube (to preserve the vacuum within it); 2) disconnect the syringe from the tube; 3) depress the syringe plunger to empty the syringe; 4) reconnect the syringe to the tube; 5) release the pinch-off; and 6) re-attempt the aspiration. This is not generally considered to be good practice because the practitioner needs to perform the operation single-handedly because one hand is occupied with pinching-off the tube. This method also has the potential to introduce a finite risk of contamination because the act of disconnection means that the components can no longer be sterile.

Secondly, the practitioner can "blow back" through the tube, by depressing the syringe plunger, to empty the syringe and try again. This also is not generally considered to be good practice because it risks inflating the body part at the distal end of the tube and in any event, puts the procedure back to the beginning.

It will be appreciated that neither of the above work-arounds are ideal, and a need therefore exists for an improved and/or an alternative type of type of syringe-based aspirator.

A first aspect of the invention provides valve assembly comprising a three-way connector and a receptacle, the three-way connector comprising and operatively interconnecting: an inlet operatively connectable, in use, to an aspirator tube, the inlet comprising a first one-way valve permitting, in use, the flow of a fluid from the tube into the three-way connector; a first outlet operatively connected, in use, to the receptacle, the first outlet comprising a second one-way valve permitting, in use, the flow of a fluid from the three-way interconnector into the receptacle; and a second outlet operatively connectable, in use, to a syringe, characterised in that the receptacle comprises a vent aperture and a porous or perforated element overlying the vent aperture, the porous or perforated element internally overlying the vent aperture and being configured to close the vent aperture when wetted by a liquid.

A second aspect of the invention provides a valve assembly comprising a three-way connector operatively interconnecting: an inlet operatively connectable, in use, to an aspirator tube, the inlet comprising a first one-way valve permitting, in use, the flow of a fluid from the tube into the three way connector; a first outlet operatively connectable, in use, to a receptacle, the first outlet comprising a second one-way valve permitting, in use, the flow of a fluid from the three-way interconnector into the receptacle; and a second outlet operatively connectable, in use, to a syringe.

A third aspect of the invention provides an aspirator comprising a tube operatively connectable, in use, via a first one-way valve, to an inlet of a three-way connector, the first one-way valve being configured to permit, in use, one-way flow of a fluid from the tube into the three-way connector; wherein the three-way connector additionally comprises a first outlet operatively connectable, in use, to a receptacle, via a second one-way valve adapted to permit, in use, the one way flow of a fluid from the three-way interconnector into the receptacle; and wherein the three-way connector further comprises a second outlet operatively connectable, in use, to a syringe.

Additionally, the aspirator of the invention suitably comprises a syringe operatively connected to the second outlet. The syringe, where provided, suitably has a capacity of less than or equal to 100ml, but preferably of less than or equal to 50ml, and most preferably, of less than or equal to 10ml, 5ml or 1ml.

By providing two one-way valves thus configured, the syringe of the invention is able to act as a pump, whereby, upon raising the plunger, fluid (gas, air or liquid) can flow into the three-way connector and into either or both of the syringe or receptacle; but on depressing the syringe's plunger, fluid within the syringe and three-way connector is forced into the receptacle, via the action of the first and second one-way valves. The invention is distinguished over the prior art by the arrangement and configuration of its valves and connectors.

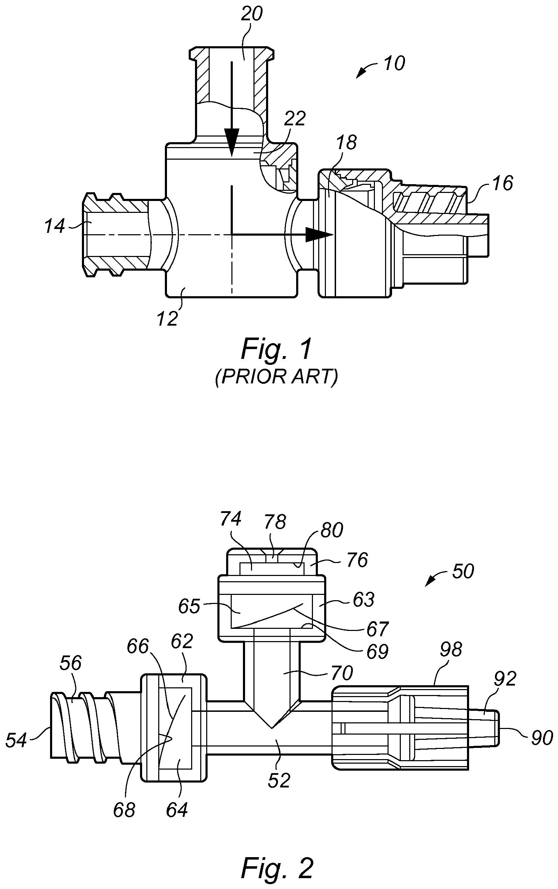

Examples of known, similar types of dual check valve that are sometimes used in infusion procedures, include Borla SpA's (IT) "4166", "4419", "4687" and "4688" anti-gravity and anti-siphon check valves, an example of which is shown in Figure 1 of the drawings.

In Figure 1, the known three-way connector 10 is used as an in-line device and comprises a main body portion 12 having a hollow interior forming a three-way connector. The connector 10 comprises an inlet port 14, to which is connected in use, a tube leading from an IV drip. The connector 10 also comprises an outlet port 16 provided with a Luer lock connector, which connects to an IV giving line. Thus, fluids are able to flow from the IV drip bag to the giving line, and thence into the patient. A one-way check valve 18 is incorporated into the outlet port 16, which prevents siphoning of body fluids back into the IV drip bag, for example if the IV drip bag is located below the patient and, for example, to enable the IV drip bag to be disconnected without blood or other body fluids flowing back up the giving line and out through the inlet port 14.

A side entry port 20 feeds into the main body portion 12 and is also provided with a second one-way check valve 22 that allows fluids to flow into the junction, but not back out of it. The side entry port 20 is also provided with a Luer connector to allow a syringe to be connected to it, so that, say liquid medication can be injected into the patient via the IV giving line. However, when the syringe (not shown) is disconnected, the second one-way check valve 22 prevents the fluid from the IV drip bag (connected to the inlet) from escaping via the side entry port 20.

The configuration of the inlet 14, outlet 16 and side entry ports 20; and the first 18 and second 22 check valves is such that the illustrated junction can only be used as an in-line device, which permits the introduction of fluids into the giving line. In fact, there is no way to connect a syringe or tubes to the known device to enable it to function to convert a syringe into a pump as does the invention.

The receptacle of the invention suitably comprises a hollow interior portion for containing a quantity of aspirated fluid and/or gas and/or air.

The receptacle of the invention may comprise a perforated wall portion that permits air or gasses to exit the receptacle, but which inhibits and/or prevents the egress of liquids from the receptacle.

The receptacle of the invention may comprise a viewing window, and/or it may be at least partially manufactured of a transparent material, to permit the contents of the receptacle to be visually inspected from the outside of it.

The receptacle of the invention may comprise an air permeable membrane which permits air to pass through it, but not fluids. The air permeable membrane is suitably manufactured of a material, such as paper, which when dry, comprises pores or perforations that allow air or gasses to pass through the material, but when wetted, for example by an aspirated liquid, the liquid closes the pores or perforations thereby preventing fluids (air and/or gas and/or liquid) to pass through it.

Such a configuration conveniently provides a self-closing vent valve that permits air or gasses to pass through it, but when the wetted, self-seals to prevent fluids from passing through it.

The air permeable membrane may be impregnated with a reagent that changes colour upon contact with a target substance. In such a situation, the air permeable membrane is suitably visible from without the receptacle, for example, through a viewing window or a transparent portion of the receptacle.

The air permeable membrane, in certain embodiments of the invention, may be manufactured from litmus paper, which changes colour on contact with acids or bases. Using litmus paper in the self-closing vent valve conveniently enables it to be used to test for the presence of stomach acid, for example, when the invention is used as an enteral aspirator or as part of an NG tube.

In an embodiment of the invention, a side wall and/or an end wall of the receptacle is at least partially manufactured of a porous, e.g. a paper-based, web of material impregnated with a testing chemical. By such a configuration, aspirated gasses may be able to pass through the web to exit the receptacle (interacting with the testing chemical as they do so), and/or aspirated liquids may be able to wet and/or pass through the web, interacting with the testing chemical as they do so.

Suitably, the testing chemical can test for the presence of a target substance, such as any one or more of the non-exhaustive list comprising: an acid, a base (alkali), carbohydrate, glucose, sugar, blood, iron, protein, ketone, bilirubin, urobilinogen, nitrates, leukocytes, etc.

A cover or cap by be provided over the web, such as a transparent plastics end cap, to seal the web from the external environment.

The tube is suitably a flexible tube, such as a plastics or rubber tube. The tube is suitably sterilisable. The tube is suitably an enteral aspirator tube or an NG tube.

The inlet and outlets of the three-way connector suitably comprise connectors for releasably connecting items thereto, such a "Luer lock" connectors, bayonet-type fittings, screw threads, push fit connectors, being either male or female. Such a configuration suitably facilitates attaching and detaching items to the three-way connector.

Suitably, the inlet of the valve assembly comprises a connector for connecting the inlet to the connector of enteral or NG tube, such as a female Luer-lock connector. Suitably, the second outlet comprises a connector suitable for connecting the second outlet to an enteral syringe, such as a male Luer or Luer-lock connector.

In an embodiment of the invention, the valve assembly effectively converts any standard syringe into a pump, allowing the piston to be pulled up and down repeatedly to pump fluid up the tube and into the receptacle. Such a configuration suitably avoids having to use an over-sized syringe to aspirate fluids and/or it suitably avoids the "disconnection-contamination", and "blowback" problems outlined above.

A further possible advantage of the invention is that it effectively converts a syringe into a pump without the need for a dedicated electric pump unit, per-se. In other words, the invention can provide an inexpensive and/or disposable alternative to, e.g. an electric pump, which electric pump would be overkill where only a small volume of sample is required.

Preferred embodiments of the invention shall now be described, by way of example only, with reference to the accompanying drawings in which:
Figure 1 is a partial lateral cross-section through a known three-way medial connector;
Figure 2 is a partial lateral cross-section through a first embodiment of a three-way valve assembly in accordance with the invention;
Figure 3 is a perspective view of an aspirator in accordance with the invention comprising the three-way valve assembly of Figure 2;
Figure 4 is a partially exploded perspective view of the aspirator of Figure 3;
Figure 5 is an exploded perspective view of the three-way valve assembly of Figures 2 to 4; and
Figure 6 is a perspective view of a second embodiment of a three-way valve assembly in accordance with the invention.

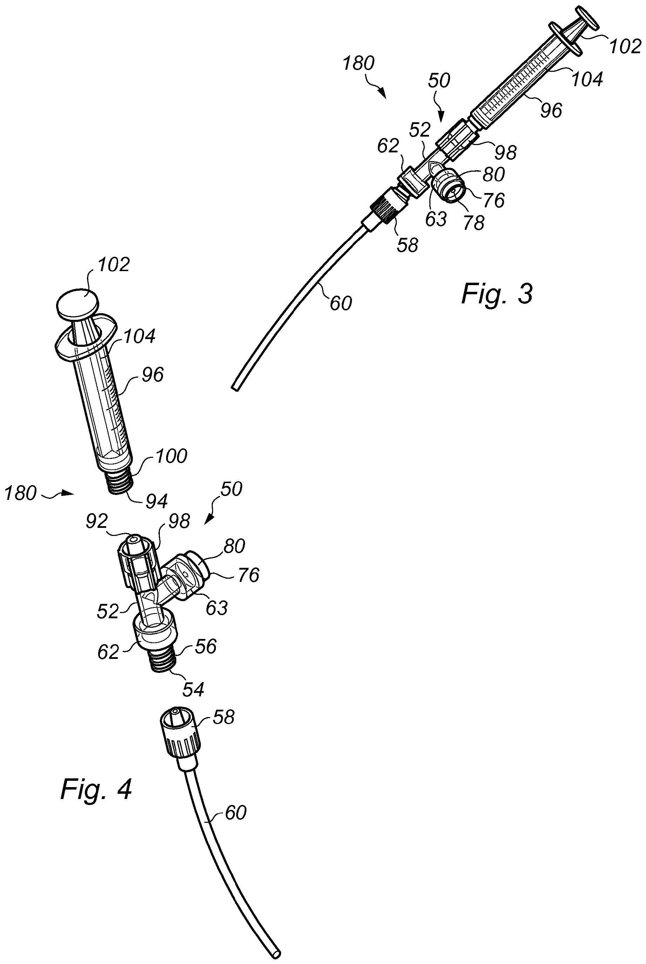

In Figures 2 to 5, a three-way valve assembly 50 in accordance with the invention comprises a main body portion 52 in the form three tubes converging to form a T-junction. The three-way valve assembly 50 has an inlet port 54 comprising a female Luer-lock connector 56 to which is connected the male Luer-lock connector 58 of an enteral or NG tube 60 in the illustrated embodiment, and which is shown in Figures 3 and 4 of the drawings.

A first one-way check valve 62 is interposed between the inlet port 54 and the main body portion 52, which check valve 62 comprises a hollow chamber 64 inside which is located a flap valve 66. The flap valve 66, under the action of fluid flowing through the chamber 64, either seats against first 68 end wall of the chamber 64 to close off the inlet port 54 and prevent fluid from passing from the main body portion 52 towards the inlet port 54, or bends away from the first end wall 68 (as shown in Figure 2) to allow fluid to pass from the inlet port 54 into the main body portion 52.

The main body portion 52 additionally comprises a first outlet 70, which is the side port of the T-junction, which leads to a receptacle 72 into which aspirated fluids are discharged.

Interposed between the main body portion 52 and the receptacle 72 is a second one-way check valve 63 which has the same basic construction and functionality as the first one-way check valve 62 previously described. The second one-way check valve 63 comprises a flap valve 67 that either seats against an end wall 69 of the chamber 65, or which bends away from the end wall 69 to permit fluids to flow from the main body portion 52 into the receptacle 72.

The receptacle 72 comprises a hollow interior volume 74, which is closed-off by a snap-on end wall portion 76, which has a vent aperture 78 therein to allow aspirated fluids to escape from it.

Located within the hollow interior volume 74 of the receptacle 74 and being arranged to overlie the vent aperture from the inside of the receptacle 72, there is provided a disc of indicator paper, such as litmus paper 80. When dry, the pores or perforations of the indicator paper 80 permit air or other gasses to be vented to atmosphere via the vent aperture 78, but when wetted by aspirated liquids, the pores and/or perforations of the indicator paper 80 are closed off, thereby closing off the vent aperture 78 and preventing or inhibiting aspirated liquids from squirting out through the vent aperture 78. Thus, the indicator paper disc 80 conveniently provides a self-closing vent valve that allows dry fluids to escape the receptacle 72, but which automatically closes-off when wetted.

Additionally, at least the end wall portion 76 of the receptacle 72 is manufactured from a transparent plastics material so that the indicator paper disc 80 can be readily inspected by a user.

Thus, colour changes in the indicator paper 80, indicating the presence or absence of certain target substances (e.g. stomach acid) can be readily determined by visual inspection.

The main body portion 52 additionally comprises a second outlet 90, which comprises a male Luer-lock connector 92 that engages with a complementary female Luer connector 94 of a syringe 96, as can be seen in Figures 3 and 4 of the drawings. An internally screw-threaded locking barrel 98 is also provided on the second outlet 90, which screw-threading engages with a complementary external screw thread 100 of the syringe tip.

The syringe 96 comprises a plunger 102 that sealingly and slidingly engages with the internal side wall of the syringe cylinder 104 in a known manner.

In use, the enteral tube 60 is fed into a body cavity and its connector 58 is connected to the connector 56 of the inlet 54 of the valve assembly 50. A syringe 96 is then connected to the second outlet 90 of the valve assembly 50 using the male 92 and female 94 connectors, and locking barrel 98 previously described. This forms an aspirator 180 in accordance with the invention, and as shown in Figure 3.

It will be noted that the aspirator 180 comprises a syringe 96 which is arranged in-line with the enteral or NG tube 60. In other words, the inlet 54 and second outlet 90 of the three-way connector 50 are located at opposite ends of the straight-through portion of the main body portion of the connector 52 with first outlet 70 being the side spur of the T junction. Such a configuration provides a number of practical advantages, compared with attaching the syringe 96 to the side spur of the T junction, namely that the syringe 96 effectively becomes an extension of the tube 60 making it much easier to manipulate and see in use, and making it less likely to become tangled with other tubes, wires etc. in the vicinity of the patient. Further, by connecting, or integrally forming, the receptacle 72 to the side spur of the T junction, it makes the receptacle 72 and/or indicator paper 80 therein more easily viewable in use: for example, the aspirator 180 can be rotated about the axis of the syringe 96 / tube 60 to orient the receptacle 72 to a preferred or optimum viewing angle. On the other hand, if the syringe were to be connected to the side spur of the T junction, then it would be necessary to flex the tube 60 to achieve a similar viewing angle, which could lead to undesirable constriction or pinching-off of the tube 60.

An operator can then repeatedly depress and withdraw the syringe plunger 102 to create a vacuum within the aspirator to draw fluids up through the enteral tube 60 on the up-stroke, and vent fluids (initially gasses) through the vent aperture 78 on the down-stroke. Eventually, a liquid may be aspirated from the body via the enteral tube 60, which liquid enters the three way connector 50 via the inlet aperture 54. Initially, the aspirated liquid may enter the syringe 96 or the receptacle 72 on the up-stroke, but on the subsequent down-stroke, the aspirated liquids will be forced into the receptacle 72 by the action of the first one-way check valve 62. The aspirated liquid will eventually wet the indicator paper 80, thus providing a diagnostic test of the liquid (e.g. a check for stomach acid) whilst at the same time, automatically closing off the vent aperture 78 by wetting the indicator paper 80 and clogging it in the manner previously described. At this point, both the first inlet valve 62 and the vent aperture 78 will be effectively "closed", thereby preventing or inhibiting further depression of the syringe plunger 102 and automatically indicating to the operator that the aspiration is complete.

The action of the first one-way check valve also provides that aspirated fluids are inhibited from, or cannot, be forced back down the enteral or NG tube 60, and therefore due to the one "way nature" of the device 50, 180, it does not necessarily need to be sterile at the point of use, although sterilisation would, of course, be desirable in certain circumstances.

The invention thus provides a convenient solution to the problem of aspirating fluids and addresses one or more of the problems outlined above.

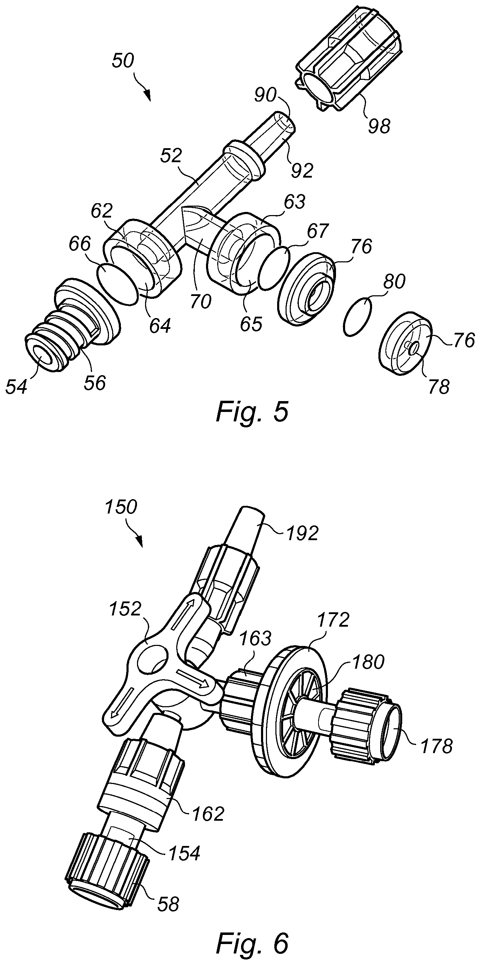

Another embodiment of the invention is shown in Figure 6 of the drawings which has been assembled from a variety of known parts to form a valve assembly for an aspirator in accordance with the invention. In the embodiment of Figure 6, the main body portion 52 of Figures 2 to 5 has been replaced by a conventional three-way flow control valve 152, set to a position with all ports "open". A first one-way check valve 162 is connected to one limb of the flow control valve 152 (i.e. at its inlet 154) to which can be connected, in use, the connector 58 of an enteral or NG tube (not shown). A second one-way check valve 163 is fitted to the side limb of the three-way valve 152 and to the outlet of the second one way check valve 163 is connected a chamber 172 containing a 19.6mm diameter disc of pH or litmus paper 180. A vent closure cap 178 is fitted to the outlet of the chamber 172. Finally, a syringe (not shown) can be connected to a Luer-lock connector 192 fitted to the third limb of the three-way control valve 152 to complete the assembly of the three-way valve assembly 150 of the invention, which can then be incorporated into an aspirator 180 in accordance with the invention.

The invention is not restricted to the details of the foregoing embodiments, which are merely exemplary of the invention. For example, any shapes, sizes, relative dimensions etc. are illustrative, and not limiting, as are any material selections and/or design choices (e.g. type of check valve).

The following statements are not the claims, but relate to various aspects and/or embodiments of the invention:

| | |
|---|---|
| Statement 1. | A valve assembly comprising a three-way connector operatively interconnecting: an inlet operatively connectable, in use, to an aspirator tube, the inlet comprising a first one-way valve permitting, in use, the flow of a fluid from the tube into the three-way connector; a first outlet operatively connectable, in use, to a receptacle, the first outlet comprising a second one-way valve permitting, in use, the flow of a fluid from the three-way interconnector into the receptacle; and a second outlet operatively connectable, in use, to a syringe. |
| Statement 2. | An aspirator comprising the valve assembly of statement 1. |
| Statement 3. | An aspirator comprising a tube operatively connectable, in use, via a first one-way valve, to an inlet of a three-way connector, the first one-way valve being configured to permit, in use, one-way flow of a fluid from the tube into the three-way connector; |
| | wherein the three-way connector additionally comprises a first outlet operatively connectable, in use, to a receptacle, via a second one-way valve adapted to permit, in use, the one-way flow of a fluid from the three-way interconnector into the receptacle; and wherein the three-way connector further comprises a second outlet operatively connectable, in use, to a syringe. |
| Statement 4. | The aspirator of statements 2 or 3, further comprising a syringe operatively connected to the second outlet. |
| Statement 5. | The aspirator of statement 4, wherein the syringe has a capacity of less than or equal to any one or more of the group comprising: 100ml; 50ml; 10ml; 5ml; and 1ml. |
| Statement 6. | The aspirator of any of statements 2 to 5, wherein upon raising the syringe's plunger, fluid flows into the three-way connector and into either or both of the syringe or receptacle, and wherein upon depressing the syringe's plunger, the first and second one-way valves cause fluid within the syringe and/or three-way connector to be directed into the receptacle. |
| Statement 7. | The valve assembly or aspirator of any preceding statement, wherein the receptacle comprises a hollow interior portion for containing a quantity of aspirated liquid. |
| Statement 8. | The valve assembly or aspirator of any preceding statement, wherein the receptacle comprises a vent aperture and a porous or perforated element overlying the vent aperture. |
| Statement 9. | The valve assembly or aspirator of any preceding statement, wherein the receptacle comprises a vent aperture and a porous or perforated element internally overlying the vent aperture and being configured to close the vent aperture when wetted by a liquid. |
| Statement 10. | The valve assembly or aspirator of statement 8 or statement 9, wherein the porous or perforated element is manufactured of paper. |
| Statement 11. | The valve assembly or aspirator of statement 10, wherein the porous or perforated element is manufactured of indicator paper. |
| Statement 12. | The valve assembly or aspirator of statement 11, wherein the indicator paper comprises a colorimetric substance that changes colour in the presence of a target substance. |
| Statement 13. | The valve assembly or aspirator of statement 12, wherein the colorimetric substance changes colour in the presence of any one or more of the group comprising: an acid; a base (alkali); a carbohydrate; glucose; sugar; blood; iron; protein; ketone; bilirubin; urobilinogen; nitrates; and leukocytes. |
| Statement 14. | The valve assembly or aspirator of any preceding statement, wherein the receptacle comprises a viewing window. |
| Statement 15. | The valve assembly or aspirator of any preceding statement, wherein the receptacle is at least partially manufactured from a transparent material. |
| Statement 16. | The valve assembly or aspirator of any of statements 11 to 15, wherein the indicator paper is visible from without the receptacle. |
| Statement 17. | The aspirator of any of statements 2 to 16, wherein the tube comprises an enteral or an NG tube. |
| Statement 18. | The valve assembly or aspirator of any preceding statement, wherein the inlet and outlets of the three-way connector comprise releasable connectors. Statement 19. The valve assembly or aspirator of statement 18, wherein the releasable connector comprises a male or female connector being any one or more of the group comprising: a Luer lock connector; a bayonet-type fittings; a screw thread; a push fit connector. |
| Statement 20. | The valve assembly or aspirator of statement 19, wherein the inlet of the valve assembly comprises a female Luer-lock connector and the second outlet comprises a male Luer or Luer-lock connector. |
| Statement 21. | The valve assembly or aspirator of any preceding statement, wherein the three-way connector is formed as a T-junction connector, the inlet and second outlet being located at opposite ends of the straight-through portion of the T-junction connector and the first outlet comprising the side spur of the T-junction. |
| Statement 22. | An enteral aspirator according to any of statements 2 to 20. |
| Statement 23. | A valve assembly or aspirator substantially as herein before described with reference to, and as illustrated in, Figures 2 to 6 of the accompanying drawings. |

## Claims

1. A valve assembly (50) comprising a three-way connector (52) and a receptacle, the three-way connector comprising and operatively interconnecting: an inlet (54) operatively connectable, in use, to an aspirator tube (60), the inlet (54) comprising a first one-way valve (66) permitting, in use, the flow of a fluid from the tube (60) into the three-way connector (52); a first outlet (70) operatively connected, in use, to the receptacle, the first outlet (70) comprising a second one-way valve (67) permitting, in use, the flow of a fluid from the three-way interconnector (52) into the receptacle (63); and a second outlet (90) operatively connectable, in use, to a syringe (96), **characterised in that** the receptacle (63) comprises a vent aperture (78) and a porous or perforated element (80) overlying the vent aperture (78), the porous or perforated element (80) internally overlying the vent aperture (78) and being configured to close the vent aperture (78) when wetted by a liquid.

2. An aspirator comprising the valve assembly (50) of claim 1.

3. The aspirator of claim 2, further comprising a tube (60) operatively connected, in use, via the first one-way valve (66), to the inlet (54) of the three-way connector (52).

4. The aspirator of claim 2 or claim 3, further comprising a syringe (96) operatively connected to the second outlet (90).

5. The aspirator of claim 4, wherein the syringe (96) has a capacity of less than or equal to any one or more of the group comprising: 100ml; 50ml; 10ml; 5ml; and 1ml.

6. The aspirator of any of claims 2 to 5, wherein upon raising the syringe's (96) plunger, fluid flows into the three-way connector (52) and into either or both of the syringe (96) or receptacle (63), and wherein upon depressing the syringe's (96) plunger, the first (66) and second (67) one-way valves cause fluid within the syringe (96) and/or three-way connector (52) to be directed into the receptacle (63).

7. The valve assembly (50) or aspirator of any preceding claim, wherein the receptacle (63) comprises a hollow interior portion for containing a quantity of aspirated liquid.

8. The valve assembly (50) or aspirator of any preceding claim, wherein the porous or perforated element (80) is manufactured of indicator paper (80) comprising a colorimetric substance that changes colour in the presence of a target substance.

9. The valve assembly (50) or aspirator of claim 8, wherein the colorimetric substance changes colour in the presence of any one or more of the group comprising: an acid; a base (alkali); a carbohydrate; glucose; sugar; blood; iron; protein; ketone; bilirubin; urobilinogen; nitrates; and leukocytes.

10. The valve assembly (50) or aspirator of any preceding claim, wherein the receptacle (63) comprises a viewing window, or wherein the receptacle (63) is at least partially manufactured from a transparent material, such that the indicator paper (80) is visible from without the receptacle (63).

11. The aspirator of any of claims 2 to 10, wherein the tube comprises an enteral or an NG tube.

12. The valve assembly (50) or aspirator of any preceding claim, wherein the inlet (54) and outlets (70, 90) of the three-way connector (52) comprise releasable connectors, the releasable connectors comprising a male or female connector being any one or more of the group comprising: a Luer lock connector; a bayonet-type fittings; a screw thread; a push fit connector.

13. The valve assembly (50) or aspirator of claim 12, wherein the inlet (54) of the valve assembly (50) comprises a female Luer-lock connector and the second outlet (90) comprises a male Luer or Luerlock connector.

14. The valve assembly (50) or aspirator of any preceding claim, wherein the three-way connector (52) is formed as a T-junction connector, the inlet and second outlet being located at opposite ends of the straight-through portion of the T-junction connector and the first outlet comprising the side spur of the T-junction.

15. An enteral aspirator according to any of claims 2 to 14.

## Patentansprüche

1. Eine Ventilbaugruppe (50), die ein Dreiwege-Verbindungsstück (52) und einen Aufnahmebehälter beinhaltet, wobei das Dreiwege-Verbindungsstück Folgendes beinhaltet und betriebsfähig miteinander verbindet: einen Einlass (54), der im Gebrauch betriebsfähig mit einem Aspiratorschlauch (60) verbunden werden kann, wobei der Einlass (54) ein erstes Einwegventil (66) beinhaltet, das im Gebrauch den Fluss eines Fluids von dem Schlauch (60) in das Dreiwege-Verbindungsstück (52) erlaubt; einen ersten Auslass (70), der im Gebrauch betriebsfähig mit dem Aufnahmebehälter verbunden ist, wobei der erste Auslass (70) ein zweites Einwegventil (67) beinhaltet, das im Gebrauch den Fluss eines Fluids von dem Dreiwege-Verbindungsstück (52) in den Aufnahmebehälter (63) erlaubt; und einen zweiten Auslass (90), der im Gebrauch betriebsfähig mit einer Spritze (96) verbunden werden kann, **dadurch gekennzeichnet, dass** der Aufnahmebehälter (63) eine Entlüftungsöffnung (78) und ein poröses oder perforiertes Element (80) beinhaltet, das über der Entlüftungsöffnung (78) liegt, wobei das poröse oder perforierte Element (80) innen über der Entlüftungsöffnung (78) liegt und dazu ausgelegt ist, die Entlüftungsöffnung (78) zu verschließen, wenn es durch eine Flüssigkeit benetzt wird.

2. Aspirator, der die Ventilbaugruppe (50) gemäß Anspruch 1 beinhaltet.

3. Aspirator gemäß Anspruch 2, der ferner einen Schlauch (60) beinhaltet, der im Gebrauch betriebsfähig über das erste Einwegventil (66) mit dem Einlass (54) des Dreiwege-Verbindungsstücks (52) verbunden ist.

4. Aspirator gemäß Anspruch 2 oder Anspruch 3, der ferner eine Spritze (96) beinhaltet, die betriebsfähig mit dem zweiten Auslass (90) verbunden ist.

5. Aspirator gemäß Anspruch 4, wobei die Spritze (96) eine Kapazität aufweist, die weniger als oder gleich einem oder mehreren aus der Gruppe ist, die Folgendes beinhaltet: 100 ml, 50 ml, 10 ml, 5 ml und 1 ml.

6. Aspirator gemäß einem der Ansprüche 2 bis 5, wobei nach dem Hochziehen des Kolbens der Spritze (96) Fluid in das Dreiwege-Verbindungsstück (52) und in die Spritze (96) oder den Aufnahmebehälter (63) oder beide hineinfließt, und wobei nach dem Niederdrücken des Kolbens der Spritze (96) das erste (66) und zweite (67) Einwegventil bewirken, dass Fluid im Innern der Spritze (96) und/oder des Dreiwege-Verbindungsstücks (52) in den Aufnahmebehälter (63) hineingeleitet werden.

7. Ventilbaugruppe (50) oder Aspirator gemäß einem der vorhergehenden Ansprüche, wobei der Aufnahmebehälter (63) einen hohlen inneren Abschnitt zum Enthalten einer aspirierten Flüssigkeitsmenge beinhaltet.

8. Ventilbaugruppe (50) oder Aspirator gemäß einem der vorhergehenden Ansprüche, wobei das poröse oder perforierte Element (80) aus Indikatorpapier (80) hergestellt ist, das eine kolorimetrische Substanz beinhaltet, die ihre Farbe ändert, wenn sie sich in Gegenwart einer Zielsubstanz befindet.

9. Ventilbaugruppe (50) oder Aspirator gemäß Anspruch 8, wobei die kolorimetrische Substanz ihre Farbe ändert, wenn sie sich in Gegenwart von einem oder mehreren aus der Gruppe befindet, die Folgende beinhaltet: eine Säure, eine Base (Lauge), ein Kohlenhydrat, Glucose, Zucker, Blut, Eisen, Protein, Keton, Bilirubin, Urobilinogen, Nitrate und Leukozyten.

10. Ventilbaugruppe (50) oder Aspirator gemäß einem der vorhergehenden Ansprüche, wobei der Aufnahmebehälter (63) ein Sichtfenster beinhaltet oder wobei der Aufnahmebehälter (63) mindestens teilweise aus einem transparenten Material hergestellt ist, so dass das Indikatorpapier (80) von außerhalb des Aufnahmebehälters (63) sichtbar ist.

11. Aspirator gemäß einem der Ansprüche 2 bis 10, wobei der Schlauch einen enteralen oder einen NG-Schlauch beinhaltet.

12. Ventilbaugruppe (50) oder Aspirator gemäß einem der vorherigen Ansprüche, wobei der Einlass (54) und die Auslässe (70, 90) des Dreiwege-Verbindungsstücks (52) lösbare Verbindungsstücke beinhalten, wobei die lösbaren Verbindungsstücke ein männliches oder weibliches Verbindungsstück beinhalten, die eines oder mehrere aus der Gruppe sein können, die Folgendes beinhaltet: ein Luer-Lok-Verbindungsstück, einen Bajonet-Verschluss, ein Schraubgewinde, ein Steckverbindungsstück.

13. Ventilbaugruppe (50) oder Aspirator gemäß Anspruch 12, wobei der Einlass (54) der Ventilbaugruppe (50) ein weibliches Luer-Lok-Verbindungsstück beinhaltet und der zweite Auslass (90) ein männliches Luer- oder Luer-Lok-Verbindungsstück beinhaltet.

14. Ventilbaugruppe (50) oder Aspirator gemäß einem der vorherigen Ansprüche, wobei das Dreiwege-Verbindungsstück (52) als ein T-Glied-Verbindungsstück ausgebildet ist, wobei der Einlass und der zweite Auslass an gegenüberliegenden Enden des gerade verlaufenden Anteils des T-Glied-Verbindungsstücks liegen und der erste Auslass die Seitenabzweigung des T-Glieds beinhaltet.

15. Enteraler Aspirator gemäß einem der Ansprüche 2 bis 14.

## Revendications

1. Ensemble vanne (50) comprenant un raccord à trois voies (52) et un réceptacle, le raccord à trois voies comprenant et interconnectant fonctionnellement : une entrée (54) pouvant être raccordée fonctionnellement, en service, à un tube d'aspirateur (60), l'entrée (54) comprenant un premier clapet anti-retour (66) permettant, en service, l'écoulement d'un fluide depuis le tube (60) dans le raccord à trois voies (52) ; une première sortie (70) raccordée fonctionnellement, en service, au réceptacle, la première sortie (70) comprenant un second clapet anti-retour (67) permettant, en service, l'écoulement d'un fluide depuis le raccord à trois voies (52) jusque dans le réceptacle (63) ; et une seconde sortie (90) pouvant être connectée fonctionnellement, en service, à une seringue (96), **caractérisé en ce que** le réceptacle (63) comprend une ouverture d'évent (78) et un élément poreux ou perforé (80) recouvrant l'ouverture d'évent (78), l'élément poreux ou perforé (80) recouvrant intérieurement l'ouverture d'évent (78) et étant configuré pour fermer l'ouverture d'évent (78) lorsqu'il est mouillé par un liquide.

2. Aspirateur comprenant l'ensemble vanne (50) selon la revendication 1.

3. Aspirateur selon la revendication 2, comprenant en outre un tube (60) raccordé fonctionnellement, en service, par l'intermédiaire du premier clapet anti-retour (66), à l'entrée (54) du raccord à trois voies (52).

4. Aspirateur selon la revendication 2 ou la revendication 3, comprenant en outre une seringue (96) raccordée fonctionnellement à la seconde sortie (90).

5. Aspirateur selon la revendication 4, dans lequel la seringue (96) a une capacité inférieure ou égale à n'importe quelle (s) valeur (s) dans le groupe consistant en : 100 ml ; 50 ml ; 10 ml ; 5 ml et 1 ml.

6. Aspirateur selon l'une quelconque des revendications 2 à 5, dans lequel à la montée du piston de la seringue (96), le fluide s'écoule dans le raccord à trois voies (52) et dans l'un ou l'autre de la seringue (96) ou du réceptacle (63) ou les deux, et dans lequel à l'enfoncement du piston de la seringue (96), les premier (66) et second (67) clapets anti-retours amènent le fluide à l'intérieur de la seringue (96) et/ou du raccord à trois voies (52) à se diriger jusque dans le réceptacle (63).

7. Ensemble vanne (50) ou aspirateur selon l'une quelconque des revendications précédentes, dans lequel le réceptacle (63) comprend une partie intérieure creuse destinée à contenir une quantité de fluide aspirée.

8. Ensemble vanne (50) ou aspirateur selon l'une quelconque des revendications précédentes, dans lequel l'élément poreux ou perforé (80) est fabriqué dans un papier indicateur (80) comprenant une substance colorimétrique qui change de couleur en présence d'une substance cible.

9. Ensemble vanne (50) ou aspirateur selon la revendication 8, dans lequel la substance colorimétrique change de couleur en présence de l'un ou plusieurs des éléments du groupe consistant en : un acide; une base (un alcalin) ; un carbohydrate ; du glucose ; du sucre ; du sang ; du fer ; une protéine ; une cétone ; la bilirubine; l'urobilinogène; des nitrates et des leucocytes.

10. Ensemble vanne (50) ou aspirateur selon l'une quelconque des revendications précédentes, dans lequel le réceptacle (63) comprend une fenêtre de visualisation, ou dans lequel le réceptacle (63) est fabriqué au moins partiellement dans un matériau transparent, de telle sorte que le papier indicateur (80) soit visible depuis l'extérieur du réceptacle (63).

11. Aspirateur selon l'une quelconque des revendications 2 à 10, dans lequel le tube comprend un tube entéral ou NG.

12. Ensemble vanne (50) ou aspirateur selon l'une quelconque des revendications précédentes, dans lequel l'entrée (54) et les sorties (70, 90) du raccord à trois voies (52) comprennent des connecteurs détachables, les connecteurs détachables comprenant un connecteur mâle ou femelle appartenant dans le groupe consistant en : un connecteur Luer-Lok ; un connecteur à baïonnette ; un pas de vis ; un connecteur à enfichage rapide.

13. Ensemble vanne (50) ou aspirateur selon la revendication 12, dans lequel l'entrée (54) de l'ensemble vanne (50) comprend un connecteur Luer-Lok femelle et la seconde sortie (90) comprend un connecteur Luer ou Luer-Lok mâle.

14. Ensemble vanne (50) ou aspirateur selon l'une quelconque des revendications précédentes, dans lequel le raccord à trois voies (52) est formé en tant que raccord à jonction en T, l'entrée et la seconde sortie étant situées à des extrémités opposées de la partie droite du raccord à jonction en T et la première sortie constituant l'embranchement latéral de la jonction en T.

15. Aspirateur entéral selon l'une quelconque des revendications 2 à 14.
